Europäisches Patentamt

European Patent Office

Office européen des brevets

(19)

(11) Numéro de publication : **0 311 541 B1**

(12) **FASCICULE DE BREVET EUROPEEN**

(45) Date de publication du fascicule du brevet :
**11.09.91 Bulletin 91/37**

(51) Int. Cl.$^5$ : **C12Q 1/06, C12Q 1/00**

(21) Numéro de dépôt : **88430025.2**

(22) Date de dépôt : **20.09.88**

(54) **Procédé de numération, de dépistage et d'identification des mycoplasmes en général et urogénitaux en particulier.**

(30) Priorité : **29.09.87 FR 8713703**
**07.01.88 FR 8800176**

(43) Date de publication de la demande :
**12.04.89 Bulletin 89/15**

(45) Mention de la délivrance du brevet :
**11.09.91 Bulletin 91/37**

(84) Etats contractants désignés :
**AT BE CH DE ES FR GB GR IT LI LU NL SE**

(56) Documents cités :
**EP-A- 0 104 001**
**EP-A- 0 160 852**
**EP-A- 0 223 685**
**FR-A- 2 176 808**
**CHEMICAL ABSTRACTS, vol. 74, no. 1, 4 janvier 1971, page 116, résumé no. 1480w, Columbus, Ohio, US; M.G. SHEPARD et al.: "Urease color test medium U-9 for the detection and identification of "T" mycoplasmas in clinical material", & APPL. MICROBIOL. 1970, 20(4), 539-43**
**CHEMICAL ABSTRACTS, vol. 87, no. 5, août 1977, page 206, résumé no. 35580n, Columbus, Ohio, US; G.K. MASOVER et al.: "The effect of growth and urea concentration on ammonia production by a urea-hydrolyzing mycoplasma (Ureaplasma urealyticum)", & J. GEN. MICROBIOL. 1977, 98(2),587-93**

(56) Documents cités :
**CHEMICAL ABSTRACTS, vol. 72, no. 10 mars 1970, page 116, résumé no. 52079p, Columbus, Ohio, US; A.W. RODWELL: "Supply of cholesterol and fatty acids for the growth of mycoplasmas", & J. GEN. MICROBIOL. 1969, 58(Pt.1), 29-37**
**CHEMICAL ABSTRACTS, vol. 100, no. 19, mai 1984, page 250, résumé no. 153210b, Columbus, Ohio, US; I. KARUBE et al.: "A catalytic immunoreactor for the amperometric determination of human serum albumin", & ANAL. CHIM. ACTA 1984, 156, 283-7**
**Applied Microbiology, Vol. 20, pages 539-543(1970)**

(73) Titulaire : **Société à Responsabilité Limitée dite: DIFFUSION BACTERIOLOGIE DU VAR - D.B.V.**
**11 place du Coquillon**
**F-83110 Sanary sur Mer (FR)**

(72) Inventeur : **Escarguel, Claude**
**11 place du Coquillon**
**F-83110 Sanary sur Mer (FR)**
Inventeur : **Grosso, Marie-Hélène**
**9 place du Coquillon**
**F-83110 Sanary sur Mer (FR)**
Inventeur : **Laconi, Patrick**
**Le Versailles 47 avenue Gambetta**
**F-83400 Hyères (FR)**

(74) Mandataire : **Rinuy, Santarelli**
**14, avenue de la Grande Armée**
**F-75017 Paris (FR)**

## EP 0 311 541 B1

## Description

La présente invention concerne un procédé de numération, de dépistage et d'identification des mycoplasmes en général et urogénitaux en particulier. Elle vise également un milieu biologique spécialement adapté à cet effet.

On sait que les mycoplasmes sont des bactéries sans paroi dotées de propriétés enzymatiques (uréase pour Ureaplasma Urealyticum et arginine décarboxylase pour Mycoplasma Hominis et Fermantans).

Ces propriétés enzymatiques ont été utilisées pour l'identification et la numération de ces souches dans les prélévements urogénitaux.

En effet, ces germes sont des bactéries commensales (présents sur des muqueuses d'individus parfaitement sains à des taux faibles inférieurs ou égaux à $10^3$ UCC/ml — unités de changement de couleur/ml-). Ils peuvent, lorsqu'une infection se déclare ou lorsqu'une muqueuse est fragilisée (infection virale, bactérienne, déséquilibre hormonal, etc.) proliférer et entraîner des états de surinfection chronique pouvant aboutir :

— soit à des stérilités tubaires ;

— soit à des stérilités masculines ;

— soit à des salpingites aigues ou chroniques ;

— soit à des syndromes d'urétroprostatite ;

— soit à des dysplasies endocervicales (lorsqu'ils surinfectent des infections virales : Papilloma Virus, Herpes, CMV, etc.).

Dans tous ces cas de figure, ils sont présents à des taux suprapathologiques supérieurs ou égaux à $10^4$ UCC/ml.

Or, jusqu'à présent, deux techniques sont utilisées pour la numération des mycoplasmes urogénitaux :

1. On numère le nombre de colonies par champ microscopique sur une gélose d'isolement.

Cette technique est assimilable à celle utilisée pour la numération des germes dans les infections urinaires (Kass).

Ce type de numération présente, cependant, l'inconvénient d'utiliser systématiquement une gélose solide dont le prix de revient est élevé, peu adapté à la recherche systématique des mycoplasmes urogénitaux.

De plus, cette technique impose une incubation en jarre anaérobie.

2. Une numération basée sur la dilution en cascade du prélèvement à analyser en milieu liquide ($U_9$ pour U. Urealyticum, $M_{42}$ pour M. Hominis) et sur le virage d'un indicateur coloré approprié du type Rouge de Phénol contenu dans le milieu de dilution.

Cette technique utilisant les propriétés enzymatiques des M. Urogénitaux présente toutefois plusieurs inconvénients :

— l'urée, en milieu liquide complexe, est instable. De ce fait, sa concentration (représentant le substrat enzymatique) peut être variable d'une série de tests à l'autre, entraînant un risque de manque de reproductibilité ;

— la lecture n'est possible qu'après une stabilité des virages de l'indicateur d'au moins 24 heures, ce qui entraîne un délai minimum de réponse de 72 heures ;

— la technique des dilutions en cascade est lourde et de plus elle est entachée d'un coefficient d'erreur dû à la manipulation ;

— on ne dispose sur le marché d'aucun "kit" contenant l'ensemble des réactifs nécessaires ainsi que des supports.

La présente invention se propose d'obvier à ces inconvénients et vise un procédé de numération et de dépistage des mycoplasmes faisant appel à la cinétique enzymatique.

On sait en effet utiliser cette dernière technique en biochimie pour doser les enzymes mais il n'a jamais été suggéré ou envisagé de l'utiliser pour le dosage des bactéries, comme c'est le cas dans le procédé de la présente invention.

La Demanderesse a en effet trouvé que la vitesse de la réponse enzymatique était proportionnelle à la quantité de Mycoplasmes présents dans l'échantillon soumis à l'analyse.

Ce procédé est essentiellement caractérisé par le fait qu'on réalise des réactions enzymatiques dans des conditions d'anaérobiose, entre un milieu liquide de croissance de mycoplasmes servant de milieu de dilution de l'échantillon du fluide à analyser et, d'une part, un premier substrat comprenant de l'urée déshydratée en présence d'un indicateur coloré de pH également sous forme déshydratée et, d'autre part, un second substrat comprenant de l'arginine également sous forme déshydratée en présence d'un indicateur coloré de pH et qu'on suit la vitesse de réponse enzymatique en notant le temps correspondant au virage des indicateurs, les quantités respectives d'urée et d'arginine, d'une part, et la concentration et les composants nutritifs dudit milieu de croissance et de dilution, d'autre part, étant préalablement choisis et étalonnés de telle sorte que, pour des Ureaplasma Urealyticum présents à des taux supra- ou infra-pathologiques, on obtienne ou non le virage des

2

EP 0 311 541 B1

indicateurs colorés au bout d'un laps de temps déterminé.

Suivant d'autres caractéristiques :

— l'indicateur de pH est choisi de manière à avoir un point de virage entre pH 6,4 et pH 8 ;

— l'indicateur de pH est avantageusement le Rouge de Phénol ;

— la concentration d'urée déshydratée et de l'indicateur de pH, d'une part, la concentration du milieu liquide de croissance et de dilution et la quantité du fluide à analyser dans ce milieu, d'autre part, sont choisies de telle sorte qu'à des taux suprapathologiques (supérieurs ou égaux à $10^4$ UCC/ml) de Ureaplasma Urealyticum, le virage de l'indicateur se produise en un laps de temps de 24 heures et qu'à des taux de U. Urealyticum infrapathologiques (inférieurs ou égaux à $10^3$ UCC/ml), le virage de l'indicateur se produise en un laps de temps de 48 heures ;

— la concentration d'arginine déshydratée et de l'indicateur de pH, d'une part, la concentration du milieu liquide de croissance et de dilution et la quantité du fluide à analyser dans ce milieu, d'autre part, sont choisies de telle sorte que l'on obtienne en 24 ou 48 heures le virage de l'indicateur pour des taux suprapathologiques (supérieurs ou égaux à $10^3$ UCC/ml) de M. Hominis et Fermantans avec absence de virage en 48 heures pour des taux infrapathologiques (inférieurs ou égaux à $10^3$ UCC/ml) ;

— le milieu de dilution de l'échantillon à analyser est essentiellement à base d'un bouillon de croissance de mycoplasmes, additionné d'éléments nutritifs appropriés ;

— le milieu de dilution de l'échantillon à analyser est avantageusement constitué de bouillon de mycoplasmes, de sérum de poulain, d'ampicilline, d'un antibiotique, d'un réducteur du type thioglycolate de Na, de levure et de cystéine.

Le procédé selon l'invention est de préférence mis en oeuvre dans des cupules de faibles dimensions renfermant les unes l'urée, les autres l'arginine déshydratée et, après y avoir introduit le liquide soumis à analyse, on réalise les conditions d'anaérobiose en recouvrant chaque cupule d'une substance huileuse inerte comme l'huile de paraffine.

Le choix des concentrations et l'étalonnage des réactifs pour la mise en oeuvre du procédé de l'invention se font en dressant une courbe de correspondance en procédant à des numérations suivant les deux techniques usuelles rappelées précédemment.

L'invention peut être mise en oeuvre, de façon simple notamment grâce à un milieu unique, ne nécessitant qu'un nombre restreint de manipulations, tout en répondant à trois critères :

— être stable et permettre sa conservation en laboratoire ainsi que son transport ;

— permettre aux mycoplasmes de conserver leur vitalité en attendant d'être mis dans leur milieu de croissance ;

— ne pas influer sur l'appréciation quantitative des résultats.

Pour concilier ces trois nécessités, la présente invention prévoit de se servir du milieu de régénération du lyophilisat comme milieu de transport de l'échantillon à analyser. En effet, les mycoplasmes étant des germes sans paroi, comme indiqué ci-dessus, donc très sensibles aux variations de pression osmotique, ils doivent être rapidement introduits dans un milieu de croissance ou de conservation. Or l'inconvénient des milieux de croissance est leur mauvaise stabilité, d'où une gestion des stocks très stricte chez les utilisateurs au niveau des centres de prélèvements. Il y a lieu de rappeler que l'inconvénient des milieux de transport est leur effet de dilution qu'ils jouent lorsque le prélèvement y est introduit avant d'être mis dans le milieu de croissance faussant totalement l'appréciation quantitative nécessaire à l'interprétation du résultat trouvé.

On entend, dans la présente demande, par milieu unique, aussi bien le milieu réactif proprement dit sous forme lyophilisée, qui est régénéré ou qui est à régénérer au moyen d'un milieu de dilution, que ce même milieu réactif stabilisé en présence de son diluant.

Ainsi, le milieu unique ci-dessus se compose

— d'une première phase dite réactive, comprenant :

● des éléments connus nécessaires à la culture des mycoplasmes du type cholestérol, extraits de levure, sérum de poulain,

● des éléments de visualisation du métabolisme des mycoplasmes du type urée, arginine ou glucose,

● un indicateur de virage (Rouge de Phénol), et

● au moins un antibiotique ;

— et d'une seconde phase, comprenant un milieu de dilution servant aussi de véhicule de l'échantillon à analyser, ce milieu étant essentiellement constitué d'un bouillon connu spécifique des mycoplasmes additionné de 1 g/1000 d'agar-agar et pouvant également contenir un antibiotique.

Suivant d'autres caractéristiques :

— le ou les antibiotiques présents dans la première phase se retrouve (ou se retrouvent) également dans la seconde phase de dilution ;

— le ou lesdits antibiotiques sont choisis parmi les antibiotiques du type ampicilline, triméthoprime et nys-

3

tatine et de préférence parmi les antibiotiques agissant sur la synthèse de l'acide folique, totalement inactif sur les mycoplasmes ;

— le milieu de dilution est exempt de tout élément instable, le rendant ainsi apte à être utilisé comme milieu de transport et de conservation d'un prélèvement donné à analyser à +4°C.

D'autres caractéristiques et les avantages de l'invention ressortiront plus clairement de la description et de l'exemple qui vont suivre :

Suivant un mode de mise en oeuvre général du procédé de l'invention, on a utilisé au moins deux cupules d'un type bien connu présentant un diamètre de 9 mm et une profondeur de 6 mm.

Dans une de ces cupules, on a introduit une quantité d'urée déshydratée de 1 mg (milligramme) et dans l'autre une quantité d'arginine déshydratée de 1,68 mg (milligramme). Dans chacune de ces cupules, on a également introduit 6,8 µg (microgramme) du même indicateur de pH déshydraté, à savoir du Rouge de Phénol.

L'échantillon prélevé sur un malade en vue d'analyse a été dilué au moyen d'un milieu désigné ci-après milieu A3 correspondant à la composition suivante et obtenue à partir d'un bouillon de mycoplasmes déshydratés (25,5 g) dissous dans 713 ml d'eau distillée. Le milieu liquide résultant a été additionné de :

— 200 ml de sérum de poulain ;
— 0,5 g d'ampicilline ;
— 8 mg de Bactrim (antibiotique) ;
— 0,5 g de réducteur (thioglycolate de sodium) ;
— 100 ml de levure ;
— 2,5 ml de cystéine à 4%.

Chacune des cupules a été recouverte de quelques gouttes d'huile de paraffine pour créer l'anaérobiose nécessaire aux réactions enzymatiques.

Cette composition et les quantités d'urée, d'arginine et d'indicateur de pH ont été adaptées en faisant des essais comparatifs utilisant les deux techniques classiques actuellement couramment utilisées, de manière à obtenir des virages en 24 heures pour des taux suprapathologiques pour U. Urealyticum et des virages en 24 ou 48 heures pour des taux suprapathologiques pour M. Hominis.

Pour ces essais comparatifs, on a utilisé, dans la technique dite de dilution, le milieu liquide de dilution classique à base d'urée et d'arginine, à raison de 150 µl de ce milieu, dans lequel on a introduit 15 µl du milieu A3 ensemencé par l'échantillon prélevé, avec dilution en cascade à raison de 15 µl de chaque prise d'essai.

Pour la technique de numération sur boite (gélose A7), on a procédé, comme cela se fait habituellement, à l'évaluation du nombre moyen de colonies de mycoplasmes par champ microscopique (avec un grossissement de 100), de la façon suivante, UFC/ml signifiant "unité formant colonie" :

```
moins de 1 colonie        : concentration de germes
                            < 10³ UFC/ml
de 1 à 5 colonies         : voisin de 10⁴ UFC/ml
de 6 à 10 colonies        : voisin de 10⁵ UFC/ml
de 11 à 20 colonies       : voisin de 10⁶ UFC/ml
supérieur à 20 colonies :        > à 10⁶ UFC/ml.
```

En procédant de la sorte à partir de prélèvements urogénitaux de patients hommes ou femmes, on a établi graphiquement la correspondance entre les différentes techniques.

Le tableau suivant donne cette correspondance.

| MALADE | NUMERATION EN MILIEU LIQUIDE | MILIEU A7 | | CUPULE UREE | CUPULE ARG |
|---|---|---|---|---|---|
| | | NB DE COL. | NUMERATION | | |
| A | $10^6$ en 96 h | > 20 | $10^6$ | Positive 24h | - |
| B | $10^3$ en 72 h | < 1 | $\leq 10^3$ | Positive 48h | - |
| C | 0 en 96 h | 0 | 0 | - | - |
| D | $10^6$ en 72 h | 12 | $10^6$ | Positive 24h | - |
| E | 0 en 96 h | 0 | 0 | - | - |
| F | $10^1$ en 72 h | 0 | 0 | - | - |
| G | contamination | 10 U | $10^5$ | Positive 24h (trouble) | Positive 24h (trouble) |
| | | > 20 M | $10^6$ | | |
| H | $10^4$ en 72 h | 3 | $10^4$ | Positive 24h | - |
| I | $10^5$ en 48 h | 7 | $10^5$ | Positive 24h | - |

On se référera aussi à la courbe figurant en annexe.

A l'examen de ce tableau et de la courbe comparative annexée, on notera que, pour des U. Urealyticum présents à des taux supérieurs ou égaux à $10^4$ UCC/ml, la cupule de l'urée change de couleur (virage de l'indicateur) en 24 heures. Le virage dans cette même cupule en 48 heures correspond soit à la présence de U. Urealyticum à un taux inférieur ou égal à $10^3$ UCC/ml (sans signification en pathologie humaine), soit à une interférence chimique ou bactérienne. L'absence de virage en 48 heures de la cupule urée permet d'affirmer l'absence totale de U. Urealyticum même à des taux faibles ($10^2$ à $10^3$ UCC/ml) étant donné la grande sensibilité des cupules d'urée.

On notera par ailleurs qu'aucune interférence (bactérienne ou chimique) ne peut fausser la réponse en 24 heures, d'où une parfaite fiabilité du dépistage qui pourra toutefois être vérifié en repiquant le milieu A3 sur une gélose solide A7 (lors d'utilisation des cupules en screening).

Pour M. Hominis et Fermantans, le pouvoir enzymatique étant moins puissant, seul un virage de la cupule d'arginine (ARG) en 48 heures permet la discrimination des taux infrapathologiques (inférieurs ou égaux à $10^3$ UCC/ml avec absence de virage de la cupule en 48 heures) des taux suprapathologiques (supérieurs ou égaux à $10^4$ UCC/ml avec virage de la cupule en 24 ou 48 heures).

Il s'ensuit que seul le virage de la cupule d'urée en 24 heures et le virage de la cupule d'arginine en 24 heures ou 48 heures sont intéressants. Le biologiste peut alors, dans ce laps de temps, affirmer s'il y a ou non une infection ou la surinfection d'une muqueuse par un mycoplasme.

En se référant au tableau ci-dessus, on constate que, pour le malade F, les résultats correspondent à une concentration qui ne semble pas être dépistée par la présente technique du fait que le seuil de sensibilité des cupules a été volontairement fixé à $10^3$ UCC/ml. Les concentrations inférieures ne présentent du reste aucun intérêt dans le diagnostic des infections à mycoplasmes. Il est du reste normal de ne pas retrouver de colonies sur le milieu A7 étant donné le facteur de dilution dû à la technique d'ensemencement direct.

Quant au malade G, on constate que seule la numération sur boîte est interprétable.

On constate alors que toutes les numérations effectuées au moyen des trois techniques en question s'inscrivent sur la droite de correspondance entre la numération sur gélose A7 et la numération par dilution et se trouvent dans la zone de la cinétique de virage lui correspondant (24 heures pour des concentrations supérieures ou égales à $10^4$ UCC/ml et 48 heures pour des concentrations inférieures à $10^3$ UCC/ml).

Comme indiqué précédemment, on constate, sur le graphique annexé, que seul le malade F ne peut s'inscrire sur cette droite.

On notera que le délai d'obtention moyen de la numération par la technique de dilution est de 72 heures, 24 heures suffisant pour des concentrations suprapathologiques, qui sont les seules intéressantes, avec la

EP 0 311 541 B1

numération en cupules selon l'invention, alors que 48 heures suffisent pour la numération sur boite.

La Demanderesse a du reste constaté que la nouvelle technique selon l'invention faisant appel à la cinétique enzymatique sur des supports contenant le substrat déshydraté donne une très bonne corrélation dans 95% des cas lorsqu'on la compare aux deux autres techniques actuellement utilisées.

Les 5% d'écart, constatés au cours de plusieurs essais peuvent être attribués :

— soit à des souches poussant préférentiellement en milieu liquide, comme c'est le cas où la numération sur gélose solide est mise en défaut ;

— soit à des souches présentant une faible activité enzymatique, comme c'est le cas où seule la numération sur boite permet une numération fiable.

On fera alors observer que le dépistage de ces 5% d'écart peut se faire en utilisant simultanément deux méthodes de numération, à savoir celle selon la présente invention et celle sur gélose solide.

Par ailleurs, la cinétique enzymatique en cupules selon l'invention, dans laquelle le substrat se trouve sous forme déshydratée, présente, par rapport à la technique de dilution en cascade, les avantages suivants :

— stabilité du substrat garantissant la reproductibilité ;

— suppression de manipulations et élimination de l'imprécision provenant de dilutions manuelles en microplaque avec des microprises d'essai ;

— délai de réponse de 24 heures au lieu de 72 heures ;

— et enfin abaissement du prix de revient lorsque l'on tient compte de tous les matériels mis en jeu.

Suivant un mode de mise en oeuvre plus particulier, on a utilisé le milieu unique entrant dans le cadre de la présente invention et défini précédemment.

L'échantillon à analyser prélevé sur un patient (prélèvement urogénital pris ici comme exemple) est introduit dans une quantité aliquote du milieu dit de dilution et conservé dans un flacon.

Si la phase active se présente sous forme lyophilisée, le milieu dit réactif est "régénéré" par addition du milieu de dilution. Bien entendu, si le milieu réactif est lui-même déjà dilué et stabilisé, il est utilisé tel quel.

Une partie aliquote du milieu contenant l'échantillon du prélèvement est versée dans une partie aliquote du milieu réactif. Le dépistage, c'est-à-dire la visualisation de la présence de la bactérie recherchée, est mis en évidence par le virage de l'indicateur (du jaune au rouge), le milieu restant par ailleurs limpide du fait que les mycoplasmes urogénitaux, à la différence des autres bactéries, ne troublent pas ledit milieu.

La numération des mycoplasmes dont la présence a ainsi été mise en évidence se fera alors par la cinétique enzymatique décrite ci-dessus. Cette technique est ainsi mise à profit dans le milieu actif (régénéré) selon l'invention pour établir un diagnostic différentiel entre les souches présentes à des taux suprapathologiques (supérieurs ou égaux à $10^3$ UCC/ml : virage en 24 heures) et celles présentes à des taux infrapathologiques (inférieurs ou égaux à $10^2$ UCC/ml : virage en 48 heures ou plus).

Dans les cas où la numération ne présente guère d'intérêt (recherche de mycoplasmes à des taux très faibles comme, par exemple dans les cas de surveillance de fécondation in vitro, d'examen de sperme, dans le cas d'hémocultures ou dans les prélèvements per-opératoires de salpingites), l'observation du virage du milieu se fera au-delà des 24 premières heures.

Dans tous les autres cas de figures, la technique utilisée pour la numération, et en particulier la technique de cinétique enzymatique, orientera le praticien sur le nombre de mycoplasmes présents dans le prélèvement.

Quant à l'identification des mycoplasmes, il est prévu, conformément à l'invention, de faire suivre les opérations de dépistage et de numération réalisées avec le milieu biologique dont il est fait état ci-dessus, d'une opération d'identification des mycoplasmes en utilisant leur profil de sensibilité aux antibiotiques (antibiogramme).

En effet, non seulement un tel antibiogramme des mycoplasmes permet de déterminer que le virage du milieu unique visé ici est bien dû à un mycoplasme et non pas à un autre germe présentant des activités enzymatiques identiques, mais en plus ledit profil trouvé permet d'affirmer de quel type, espèce ou genre il s'agit. La mise en oeuvre de cette phase d'identification se fera sur une même galerie regroupant des caractères d'identification et des antibiotiques à tester grâce à une seconde fraction aliquote d'un autre milieu réactif identique au premier auquel on ajoute le milieu précédemment utilisé, aussitôt le virage de l'indicateur constaté, milieu toujours limpide (24 heures par exemple pour les prélèvements urogénitaux classiques, urètre, vagin, endocol et au-delà de 24 heures pour les autres cas cités ci-dessus).

On observera et notera alors le profil de cette galerie comme cela est bien connu dans la technique, ce qui permettra de différencier Ureaplasma de Mycoplasma (Hominis ou fermantans) et d'apprécier la sensibilité de la souche isolée aux antibiotiques présents dans ladite galerie (résultats de 24 à 48 heures).

On voit ainsi qu'avec un milieu unique, complété par une galerie d'antibiogramme spécifique, il est possible de réaliser, de 48 à 72 heures, le transport d'un prélèvement, le dépistage, l'appréciation quantitative, l'identification et la sensibilité aux antibiotiques, en particulier de plus de 98% des souches de mycoplasmes urogénitaux responsables d'infections (Mycoplasma fermantans représente moins de 2% de la pathologie ; il est

6

toutefois dépisté, mais assimilé à Hominis par son caractère arginine +).

L'intérêt du milieu selon l'invention réside donc dans :

1. La simplicité de la technique.

2. L'absence de faux positif (les contaminants expriment rarement leurs caractères enzymatiques en 24 heures ; au-delà, le milieu semi-gélosé permettra de caractériser par le trouble la présence d'éventuels contaminants résistant à l'association ampicilline, triméthoprime et nystatine).

Si toutefois certaines souches sensibles au mélange antibiotiques présent dans le milieu de l'invention (liquide restant clair) expriment leur caractère enzymatique urée ou arginine + (bouillon rouge limpide), il sera impossible de rendre un résultat faussement positif, étant donné le profil aberrant que présentera la galerie antibiogramme et l'éventuel virage de la cupule "contaminants" présente sur cette galerie.

3. La possibilité d'avoir un milieu de transport stable (séparation des facteurs labiles présents dans le lyophilisat).

4. La possibilité d'introduire systématiquement dans le diagnostic des mycoplasmes une étude de sensibilité aux antibiotiques qui est d'autant plus justifiée que 15% de souches sont résistants aux tétracyclines et que plus de 30% d'ureaplasma urealyticum et 97% de mycoplasma hominis sont résistants à l'érythromycine (Congrès I.C.A.A.C. New York 1987).

5. La possibilité de diagnostiquer de façon précise les 10% de souches poussant préférentiellement en milieu liquide, qu'il était impossible jusqu'à présent de différencier d'éventuels contaminants (urée ou arginine + et sensibles aux antibiotiques présents dans le milieu : milieux limpides ayant viré au rouge, exemple : campylobacter, certains anaérobies ou micro-aérophiles de la flore vaginale). Ces 10% de souches ne poussant pas sur les géloses d'isolement solides, le diagnostic était toujours délicat.

6. La possibilité d'éliminer totalement les conditions d'anaérobiose en jarres ou en sachets, relativement coûteux et parfois délicats à mettre en oeuvre pour des laboratoires non spécialisés.

Il va de soi que la présente invention n'a été décrite qu'à titre purement explicatif et nullement limitatif et que toute modification utile pourra y être apportée au niveau des équivalences sans sortir de son cadre.

C'est ainsi en particulier que le milieu unique selon l'invention peut aussi être utilisé pour le diagnostic des mycoplasmes pneumoniers (dont les pneumopathies atypiques) moyennant l'utilisation du glucose à la place de l'urée et de l'arginine et d'un indicateur approprié (Bleu de Thymol).

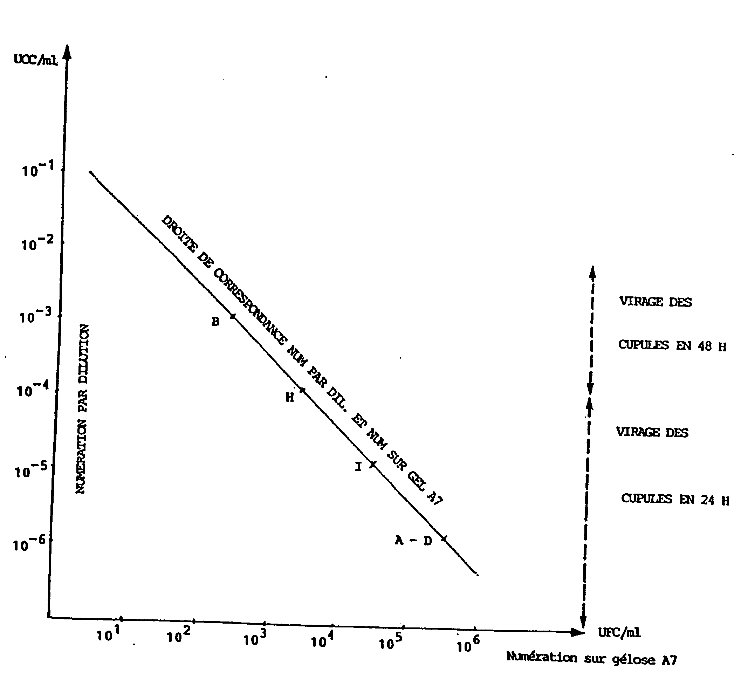

## Revendications

1. Procédé de numération et de dépistage des mycoplasmes caractérisé en ce que l'on réalise des réactions enzymatiques dans des conditions d'anaérobiose, entre un milieu liquide de croissance de mycoplasmes servant de milieu de dilution de l'échantillon du fluide à analyser, et

— un premier substrat comprenant de l'urée déshydratée en présence d'un indicateur coloré de pH également sous forme déshydratée ;

— un deuxième substrat comprenant de l'arginine également sous forme déshydratée en présence d'un indicateur coloré de pH, et si désiré,

— un troisième substrat contenant du glucose en présence d'un indicateur coloré de pH également sous forme déshydratée,

que l'on suit la vitesse de réponse enzymatique en notant le temps correspondant au virage des indicateurs, les quantités respectives d'urée et d'arginine, la concentration et les composants nutritifs dudit milieu de croissance et de dilution étant préalablement choisis et étalonnés de telle sorte que pour respectivement des Ureaplasma urealyticum et des Mycoplasma hominis ou fermantans ou des mycoplasmes pneumoniers présents à des taux supra- ou infrapathologiques, on obtienne ou non le virage des indicateurs colorés au bout d'un laps de temps déterminé.

2. Procédé selon la revendication 1, caractérisé en ce que l'indicateur de pH est choisi de manière à avoir un point de virage entre pH 6,4 et pH 8.

3. Procédé selon la revendication 2, caractérisé en ce que l'indicateur de pH est le Rouge de Phénol ou le Bleu de Thymol.

4. Procédé selon l'une quelconque des revendications 1 à 3, caractérisé en ce que la concentration d'urée déshydratée et de l'indicateur de pH d'une part, la concentration du milieu liquide de croissance et de dilution et la quantité de fluide à analyser dans ce milieu, d'autre part, sont choisies de telle sorte qu'à des taux suprapathologiques (supérieurs ou égaux à $10^4$ UCC/ml) de Ureaplasma urealyticum, le virage de l'indicateur se produise en un laps de temps de 24 heures et qu'à des taux de U. urealyticum infrapathologiques (inférieurs ou égaux à $10^3$ UCC/ml), le virage de l'indicateur se produise en un laps de temps de 48 heures.

5. Procédé selon l'une quelconque des revendications 1 à 3, caractérisé en ce que la concentration d'arginine déshydratée et de l'indicateur de pH d'une part, la concentration du milieu liquide de croissance et de dilution et la quantité du fluide à analyser dans ce milieu, d'autre part, sont choisies de telle sorte que l'on obtienne en 24 ou 48 heures le virage de l'indicateur pour des taux suprapathologiques (supérieurs ou égaux à $10^3$ UCC/ml) de M. hominis et fermantans avec absence de virage en 48 heures pour des taux infrapathologiques (inférieurs ou égaux à $10^3$ UCC/ml).

6. Procédé selon l'une quelconque des revendications 1 à 5, caractérisé en ce que le milieu de dilution de l'échantillon à analyser est à base d'un bouillon de croissance de mycoplasmes, additionné d'éléments nutritifs appropriés.

7. Procédé selon la revendication 6, caractérisé en ce que le milieu de dilution de l'échantillon à analyser est constitué de bouillon de mycoplasmes, de sérum de poulain, d'ampicilline, d'un antibiotique, d'un réducteur du type thioglycolate de Na, de levure et de cystéine.

8. Procédé selon l'une quelconque des revendications 1 à 7, caractérisé en ce qu'il est mis en oeuvre dans des cupules de faibles dimensions renfermant les unes, l'urée ou le glucose, les autres l'arginine déshydratée ou le glucose et, qu'après avoir introduit le liquide soumis à analyse, on réalise les conditions d'anaéorobiose en recouvrant chaque cupule d'une substance huileuse inerte comme l'huile de paraffine.

9. Procédé selon l'une quelconque des revendications 1 à 8, caractérisé en ce que le choix des concentrations et l'étalonnage des réactifs pour la mise en oeuvre de ce procédé se font en dressant une courbe de correspondance en procédant à des numérations suivant les techniques usuelles.

10. Application du procédé selon l'une quelconque des revendications 1 à 9 au dépistage, à la numération de tous mycoplasmes quel que soit leur type, espèce ou genre.


## Patentansprüche

1. Verfahren zum Aufzählen und Nachweisen von Mykoplasmen, dadurch gekennzeichnet, daß enzymatische Reaktionen unter Anaerobiosebedingungen zwischen einem flüssigen Wachstumsmedium von Mykoplasmen, das als Verdünnungsmedium der Probe der zu untersuchenden Flüssigkeit dient, und

einem ersten Substrat umfassend dehydratisierten Harnstoff in Anwesenheit eines pH-Farbindikators, ebenfalls in dehydratisierter Form,

einem zweiten Substrat umfassend Arginin, ebenfalls in dehydratisierter Form, in Anwesenheit eines pH-Farbindikators, und, wenn gewünscht,

einem dritten Substrat enthaltend Glukose in Anwesenheit eines pH-Farbindikators,ebenfalls in dehydratisierter Form, durchgeführt werden und daß die Geschwindigkeit der enzymatischen Reaktion durch Aufzeichnen der dem Umschlagen der Indikatoren entsprechenden Zeiträume, der jeweiligen Mengen an Harnstoff und

Arginin, der Konzentration und der Nährbestandteile des Wachstumsund Verdünnungsmediums, die vorher derart ausgewählt und abgeglichen wurden, daß jeweils für Ureaplasma urealyticum und Mycoplasma hominis oder fermantans oder Mycoplasma pneumoniae, die in supra- oder infrapathologischen Graden vorhanden sind, das Umschlagen der Farbindikatoren am Ende eines bestimmten Zeitraumes erhalten wird oder nicht, verfolgt wird.

2. Verfahren nach Anspruch 1, dadurch gekennzeichnet, daß der pH-Indikator so ausgewählt ist, daß ein Umschlagpunkt zwischen pH 6,4 und pH 8 erhalten wird.

3. Verfahren nach Anspruch 2, dadurch gekennzeichnet, daß der pH-Indikator Phenolrot oder Thymolblau ist.

4. Verfahren nach einem der Ansprüche 1 bis 3, dadurch gekennzeichnet, daß einerseits die Konzentration von dehydratisiertem Harnstoff und dem pH-Indikator und andererseits die Konzentration des flüssigen Wachstums- und Verdünnungsmediums und die Menge der zu untersuchenden Flüssigkeit in diesem Medium derart ausgewählt sind, daß bei suprapathologischen Graden (von mehr als oder gleich $10^4$ UCC/ml) von Ureaplasma urealyticum das Umschlagen des Indikators in einem Zeitraum von 24 Stunden erfolgt und daß bei infrapathologischen Graden von U. urealyticum (von weniger als oder gleich $10^3$ UCC/ml) das Umschlagen des Indikators in einem Zeitraum von 48 Stunden erfolgt.

5. Verfahren nach einem der Ansprüche 1 bis 3, dadurch gekennzeichnet, daß einerseits die Konzentration von dehydratisiertem Arginin und dem pH-Indikator und andererseits die Konzentration des flüssigen Wachstums- und Verdünnungsmediums und die Menge der zu untersuchenden Flüssigkeit in diesem Medium derart ausgewählt sind, daß in 24 oder 48 Stunden das Umschlagen des Indikators bei suprapathologischen Graden (von mehr als oder gleich $10^3$ UCC/ml) von M. hominis und fermantans bei Fehlen des Umschlagens in 48 Stunden bei infrapathologischen Graden (von weniger als oder gleich $10^3$ UCC/ml) erhalten wird.

6. Verfahren nach einem der Ansprüche 1 bis 5, dadurch gekennzeichnet, daß das Verdünnungsmedium der zu untersuchenden Probe auf einer Wachstumsbrühe von Mykoplasmen, der Nährstoffe zugesetzt wurden, basiert.

7. Verfahren nach Anspruch 6, dadurch gekennzeichnet, daß das Verdünnungsmedium der zu untersuchenden Probe aus Brühe von Mykoplasmen, Fohlenserum, Ampicillin, einem Antibiotikum, einem Reduktionsmittel des Typs Natriumthioglykolat, Hefe und Cystein besteht.

8. Verfahren nach einem der Ansprüche 1 bis 7, dadurch gekennzeichnet, daß es in Cupulae mit geringen Abmessungen, wobei die einen Harnstoff oder Glukose und die anderen dehydratisiertes Arginin oder Glukose enthalten, durchgeführt wird und daß nach dem Einbringen der der Analyse unterworfenen Flüssigkeit die Anaerobiosebedingungen durchgeführt werden, wobei jede Cupula mit einer inerten öligen Substanz, wie Paraffinöl, überzogen wird.

9. Verfahren nach einem der Ansprüche 1 bis 8, dadurch gekennzeichnet, daß die Auswahl der Konzentrationen und das Abgleichen der Reaktionsmittel zur Durchführung des Verfahrens durch Zeichnen einer Entsprechungskurve erfolgt, wobei die Zählungen gemäß den üblichen Techniken vorgenommen werden.

10. Anwendung des Verfahrens nach einem der Ansprüche 1 bis 9 zum Nachweisen und Zählen aller Mykoplasmen, sei es ihres Typs, ihrer Art oder Gattung.


## Claims

1. Method for the detection and numeration of mycoplasmas wherein enzymatic reactions are carried out under anaerobic conditions, between a liquid mycoplasma growth medium also used as dilution medium for the sample to be analyzed, and :

— a first substrate comprising dehydrated urea in the presence of a coloured pH indicator also dehydrate;

— a second substrate comprising also dehydrated arginine in the presence of a coloured pH indicator, also dehydrated and, if desired,

— a third substrate comprising glucose in the presence of a coloured pH indicator, both also dehydrated, and wherein the rate of the enzymatic response is followed while noting the delays corresponding to the colour change of the pH indicators, the respective amounts of urea and arginine, the concentration and the nutritional elements of the aforementioned growth and dilution medium having been beforehand chosen and standardised such that, for U. urealyticum and M. hominis or M. fermentans, or pulmonary mycoplasmas present at supra- or subpathological levels, a colour change of the pH indicators is, or not, obtained within a predetermined lapse of time.

2. Method according to claim 1, characterised by the fact that the pH indicator is chosen such that it will have a colour change point between pH 6.4 and pH 8.

3. Method according to claim 2, characterised by the fact that the pH indicator is either Phenol Red or Thymol Blue.

4. Method according to anyone of claims 1 to 3, characterised by the fact that the concentration of dehydrated urea and of the pH indicator on one hand, the concentration of growth and dilution liquid medium and the quantity of fluid to be analysed in this medium on the other hand, are defined such that, at suprapathological levels of U. urealyticum ($\geq 10^4$ CCU/ml), the colour change of the pH indicator occurs within 24 hours and that at subpathological levels of U. urealyticum ($\leq 10^3$ CCU/ml), the colour change of the pH indicator occurs within 48 hours.

5. Method according to anyone of claims 1 to 3, characterised by the fact that the concentration of dehydrated arginine and of the pH indicator on one hand, the concentration of growth and dilution liquid medium and the quantity of fluid to be analysed in this medium on the other hand, are chosen such that a colour change of the pH indicator occurs within 24 to 48 hours for suprapathological levels of M. hominis and M. fermentans ($\geq 10^3$ CCU/ml) and that no colour change occurs within 48 hours for subpathological levels ($\leq 10^3$ CCU/ml).

6. Method according to anyone of claims 1 to 5, characterised by the fact that the dilution medium for the sample to be analysed is based on a mycoplasma growth broth, supplemented with appropriate nutritional elements.

7. Method according to claim 6, characterised by the fact that the dilution medium for the sample to be analysed is comprised of mycoplasma broth, foal serum, ampicillin, an antibiotic, a reducing agent of sodium thioglycolate tyre, yeast and cysteine.

8. Method according to anyone of claims 1 to 7, characterised by the fact that it is carried out in wells of small dimensions, the ones enclosing either urea or glucose, the others the dehydrated arginine or the glucose and that, after having added the liquid to be analysed, anaerobic conditions are established by covering each well with an inert oily substance such as paraffin oil.

9. Method according to anyone of claims 1 to 8, characterised by the fact that the choice of the concentrations and the standardisation of the reagents for the implementation of this method are obtained by establishing a correlation curve using numerations according to conventional techniques.

10. Use of the process according to anyone of claims 1 to 9, for the detection and numeration of all mycoplasmas, regardless of type, species or genus.